# EUROPEAN PATENT APPLICATION

(11) **EP 2 384 735 A2**
(43) Date of publication of application: **09.11.2011**
(21) Application number: 11380006.4
(22) Date of filing: 28.01.2011
(51) Int. Cl.: A61K 8/02, A61K 8/11, A61K 8/19, A61K 8/26, A61Q 19/00

(54) **Cosmetic composition containing powder particles**

(30) Priority: 29.01.2010 ES 201030113
(71) Applicant: Hilatura Científica Atais, S.L., 46116 Masias-Moncada (Valencia) (ES)
(72) Inventor: Portales Reig, José Vicente, 46100 Burjassot (Valencia) (ES)
(74) Representative: Ungria Lopez, Javier

(57) **Abstract**

The present invention relates to a cosmetic composition characterized in that it comprises powder particles of at least one component selected from the group consisting of: aluminum, ferrite, an iron oxide, an iron hydroxide, an iron oxyhydroxide, a crystalline phase found in steels, and any combination thereof. Said particles that may be present in the composition in very different percentages, sizes and forms (e.g., encapsulated), provide the cosmetic composition the property of refracting and attenuating the electromagnetic waves, especially radiofrequencies. Preferably, the cosmetic composition is for topical use and is intended for skin care, preferably being cream, milk and body gel with multiple uses. Accordingly, object of the present invention is the use of the powder particles of the cited components in order to obtain a cosmetic composition with the mentioned property, as well as the topical use of said composition for skin care.

## Description

### TECHNICAL FIELD

The present invention falls within the field of cosmetics. In particular, it relates to cosmetic products such as those of topical use (moistening, sun, makeup cream body...) comprising in their composition powder particles of certain metallic components, such as aluminum, derivatives of iron and steel, said products being preferably designed for skin care.

### DESCRIPTION OF THE INVENTION

The present invention relates to a cosmetic composition (or formulation) characterized in that it comprises powder particles of at least one component selected from the group consisting of: aluminum, ferrite, iron oxide, iron hydroxide, iron oxyhydroxide, a crystalline phase found in steel and any combination thereof. It has been found that the incorporation of powder particles of the listed elements and compounds provides said cosmetic compositions the property of refracting and attenuating the electromagnetic waves, among them radiofrequency waves.

In a preferred embodiment, the iron oxide is selected from the group consisting of hematite (α-Fe₂O₃), maghemite (γ-Fe₂O₃) and magnetite (Fe₃O₄). In another preferred embodiment, the iron hydroxide is bernalite (Fe(OH)₃. Also in another preferred embodiment, the iron oxyhydroxide is selected from the group consisting of goethite (α-FeO·OH), akaganeite (β-FeO·OH), lepidocrocite (γ-FeO·OH), feroxyhite (δ-FeO·OH) and ferrihydrite (Fe₅HO₈·4H₂O, approximately). In another embodiment, the crystalline phase found in steels is selected from the group consisting of martensite, austenite, pearlite and bainite. As it is derived from previous comments, the cosmetic composition object of the invention comprises powder particles of only one of the listed component or of any combination thereof.

Preferably, aluminum powder particles have a grain size between 0.40495 nanometers and 100 microns, both boundaries included, a particle size of 0.40495 nanometers corresponds to the size wherein the aluminum crystallizes. Preferably, aluminum powder particles have a particle size between 0.01 microns and 100 microns, both boundaries included.

Powder aluminum can be presented in the following ways: powder aluminum nanoparticles, atomized aluminum powder, spherical aluminum powder, aluminum gas atomized powder aluminum nanoparticles, air atomized in aluminum powder, irregular powder, flaked aluminum powder, aluminum granules, aluminum metal powder, aluminum crystals, objective aluminum for cathodic pulverization, and a combination thereof.

In turn, the grain size of the powder particles of ferrite, iron oxide, iron hydroxide, iron oxyhydroxide and crystalline phase found in steels is preferably between 50 nanometers and 1000 microns, both boundaries included. The powder particles of these compounds can be presented in forms similar to those of the aluminum powder: powder nanoparticles, atomized powder, spherical powder, gas atomized powder nanoparticles, powder atomized air, irregular powder, flaked powder, granules, crystals, objective powder for cathodic pulverization and a combination thereof.

Preferably, the powder particles are present in the cosmetic composition in a concentration between 1% and 75% of the total composition, both boundaries included.

In a preferred embodiment, the powder particles of the mentioned metallic elements and compounds are capsulated. Said encapsulation consists in that each particle is trapped inside and isolated from the outside by using conventional industrial processes creating isolation. The powder particles are encapsulated (microencapsulated) in at least one inert compound selected from an organic compound, an inorganic compound and any combination thereof. Preferably, the at least one microencapsulated inert compound is selected from paraffin, a resin, a plasticizer compound or any synthetic material accepting the encapsulation process.

Preferably, the cosmetic composition comprising powder particles of the listed metallic elements and compounds, in all variations and embodiments discussed here it is for topical use and is intended for skin care, presenting in one of the forms selected from the group consisting of body cream, body milk and body gel for multiple applications. More preferably, said cosmetic composition is presented in one of the following forms: moistening body cream, sun body cream, makeup body cream, tanning body cream, anti-wrinkle body cream, moistening body milk, sun body milk, makeup body milk, tanning body milk, anti-wrinkle body milk, moistening body gel, solar body gel, makeup body gel, tanning body gel and anti-wrinkle body gel. The addition of powder particles of the metallic elements and compounds described above provides to such products the property of refracting and attenuating electromagnetic waves, especially radiofrequency waves.

Another object of this invention is the use of powder particles of at least one component selected from the group consisting of aluminum, ferrite, iron oxide, iron hydroxide , iron oxyhydroxide, a crystalline phase found in steels and any combination thereof for manufacturing a cosmetic composition as described herein, in all its variations and alternatives. More preferably, the iron oxide is selected from the group consisting of hematite (α-Fe₂O₃), maghemite (γ-Fe₂O₃) and magnetite (Fe₃O₄). In another preferred embodiment, the iron hydroxide is bernalite (Fe(OH)₃. Also in another preferred embodiment, the iron oxyhydroxide is selected from the group consisting of goethite (α-FeO·OH), akaganeite (β-FeO·OH), lepidocrocite (γ-FeO·OH), feroxyhite (δ-FeO·OH) and ferrihydrite (Fe₅HO₈·4H₂O, approximately). In another embodiment, the crystalline phase found in steels is selected from the group consisting of martensite, austenite, pearlite and bainite.

More preferably, the powder particles in question are used for producing and manufacturing body cream, body milk and body gel with multiple applications.

Preferably, the cosmetic composition comprising powder particles of the constituents listed in this specification (elements such as aluminum and compounds derived from iron and steel) is for topical use and is intended for skin care.

## Claims

1. Cosmetic composition **characterized in that** it comprises powder particles of at least one component selected from the group consisting of aluminum, ferrite, an iron oxide, an iron hydroxide iron, an iron oxyhydroxide, a crystalline phase found in steel and any combination thereof.

2. Cosmetic composition according to claim 1, **characterized in that** the iron oxide is selected from the group consisting of hematite, maghemite and magnetite.

3. Cosmetic composition according to any one of claims 1 or 2, **characterized in that** the iron hydroxide is bernalite.

4. Cosmetic composition according to any one of claims 1 to 3, **characterized in that** the iron oxyhydroxide is selected from the group consisting of goethite, akaganeite, lepidocrocite, feroxyhite and ferrihydrite.

5. Cosmetic composition according to any one of claims 1 to 4, **characterized in that** the crystalline phase found in steel is selected from the group consisting of martensite, austenite, pearlite and bainite.

6. Cosmetic composition according to any one of claims 1 to 5, **characterized in that** the aluminum powder particles have a grain size between 0.40495 nanometers and 100 microns, both boundaries included.

7. Cosmetic composition according to any one of claims 1 to 6, **characterized in that** aluminum powder particles have a grain size comprised between 0.01 microns and 100 microns, both boundaries included.

8. Cosmetic composition according to any one of preceding claims, **characterized in that** the powder particles of ferrite, iron oxide, iron hydroxide, iron oxyhydroxide and crystalline phase found in steels have a grain size comprised between 50 nanometers and 1000 microns, both boundaries included.

9. Cosmetic composition according to any one of preceding claims, **characterized in that** the powder aluminum particles are presented in one of the forms selected among: powder aluminum nanoparticles, atomized aluminum powder, spherical aluminum powder, aluminum gas atomized powder aluminum nanoparticles, air atomized in aluminum powder, irregular powder, flaked aluminum powder, aluminum granules, aluminum metal powder, aluminum crystals, objective aluminum for cathodic pulverization, and a combination thereof.

10. Cosmetic composition according to any one of preceding claims, **characterized in that** the powder particles of ferrite, iron oxide, iron hydroxide, iron oxyhydroxide and crystalline phase found in steels are presented in one of the forms selected among: powder nanoparticles, atomized powder, spherical powder, gas atomized powder nanoparticles, powder atomized air, irregular powder, flaked powder, granules, crystals, objective powder for cathodic pulverization and a combination thereof.

11. Cosmetic composition according to any one of preceding claims, **characterized in that** the powder particles are presented in a concentration between 1% and 75% of the total composition, both boundaries included.

12. Cosmetic composition according to any one of preceding claims, **characterized in that** the powder particles are microencapsulated in at least one inert compound selected from an organic compound, an inorganic compound and any combination of both.

13. Cosmetic composition according to claim 12, **characterized in that** the at least one microencapsulated inert compound is selected from a paraffin, a resin, a plasticizer compound.

14. Cosmetic composition according to any one of preceding claims, **characterized in that** it is presented in one form selected from the group consisting of: body cream, body milk and body gel.

15. Cosmetic composition according to any one of preceding claims, **characterized in that** it is presented in one form selected from the group consisting of: moistening body cream, sun body cream, makeup body cream, tanning body cream, anti-wrinkle body cream, moistening body milk, sun body milk, makeup body milk, tanning body milk, anti-wrinkle body milk, moistening body gel, solar body gel, makeup body gel, tanning body gel and anti-wrinkle body gel.

16. Use of the powder particles of at least one of the components selected from the group consisting of aluminum, ferrite, an iron oxide, an iron hydroxide, an iron oxyhydroxide, a crystalline phase found in steels and any combination thereof for manufacturing a cosmetic composition as described in any one of claims 1 to 15.

17. Use according to claim 16 for manufacturing a cosmetic composition in one of the forms selected from the group consisting of: body cream, body milk and gel milk.

18. Use of the cosmetic composition described in any one of claims 1 a 15 for skin care.
